# EUROPEAN PATENT SPECIFICATION

(11) **EP 0 636 184 B1**
(45) Date of publication and mention of the grant of the patent: **30.08.2000**
(21) Application number: 94906585.8
(22) Date of filing: 11.01.1994
(51) Int. Cl.: C12Q 1/24, A01N 1/02, A61F 2/06, A61L 27/00

(54) **MEDIA FOR ISOLATION AND STABILIZATION OF CELLS**
MEDIEN FÜR DIE ISOLIERUNG UND STABILISIERUNG VON ZELLEN
MILIEUX POUR L'ISOLATION ET LA STABILISATION DE CELLULES

(30) Priority: 15.01.1993 US 6108
(43) Date of publication of application: 01.02.1995
(73) Proprietor: BAXTER INTERNATIONAL INC., Deerfield, IL 60015-4633 (US)
(72) Inventor: HU, Can, B., Irvine, CA 92614 (US); NORTHUP, Sharon, J., Kildeer, IL 60047 (US); MYERS, Keith, E., El Toro, CA 92630 (US); QUIJANO, Rodolfo, C., Laguna Hills, CA 92653 (US)
(74) Representative: MacGregor, Gordon
(86) International application number: US9400352
(87) International publication number: WO9416099

(56) References cited:
- EP-A- 0 444 270
- EP-A- 0 446 450
- TRANSFUSION vol. 31, no. 1 , 1991 pages 21 - 25 G.ROCK ET AL. 'Storage of platelets in balanced salt solutions: a simple platelet storage medium'
- ANNALS OF SURGERY vol. 194, no. 2 , August 1981 pages 199 - 211 F.G.BAUMANN ET AL. 'Vein Contraction and Smooth Muscle Cell Extensions as Causes of Endothelial Damage during Graft Preparation'

## Description

### BACKGROUND OF THE INVENTION

### Field of the Invention

The present invention relates generally to the isolation and stabilization of animal cells. In one of its more particular aspects this invention relates to the use of such cells in surgical procedures.

### Description of Relevant Art

Living animal cells find extensive use in surgical procedures. Such cells are used, for example, in organ transplant surgery, in the providing of artificial organs and members, and in vascular graft surgery. A wide variety of animal cell types have been utilized in various surgical procedures. Illustrative of the cell types which have been used are hepatocytes, pancreatic islets, adipocytes, endothelial cells, and myocytes. Cells of the foregoing types and other types of cells typically have been isolated from blood vessels, organs and other animal tissues.

In the case of vascular grafts, particularly synthetic vascular grafts, viable microvessel endothelial cells are used to prevent thrombosis. Thrombosis in the vascular graft, a serious problem encountered in surgery, can be prevented by coating a layer of viable microvessel endothelial cells inside a synthetic or natural graft.

Microvessel endothelial cells are available from a variety of sources. One widely used source is fatty tissue from which microvessel endothelial cells can be isolated by means of various techniques. Isolation can be achieved mechanically, chemically or enzymatically. For example, dissociation or digestion of fatty tissue can be accomplished by the use of proteolytic enzymes. Collagenase, dispase, trypsin, elastase, papain, chymopapain, pronase, hyaluronidase and combinations thereof are particularly useful for dissociating the complex mixtures of proteins, glycoproteins, lipids, glycolipids and mucopolysaccharides found in connective tissue. The major proteins present in animal tissue form a matrix for holding cells together. These proteins include collagen, elastin and fibronectin, which are interwoven in a hydrated gel formed by a network of glycosaminoglycan chains.

Enzymatic means have been widely used to digest animal tissues in order that the desired cells can be isolated from the tissue and used, for example, in vascular grafts. Other frequently used means for isolating cells from tissue include mechanical means, such as cutting, mincing, shearing, sieving or scratching adherent cells from the tissue with a rubber policeman, and chemical means, such as divalent ion deprivation or the use of chelating agents.

Recently, it has been found convenient of use various enzymes in procedures involving the dissociation of fatty tissue and the isolation of microvessel endothelial cells embedded in such fatty tissue. These procedures generally involve mixing fatty tissue having embedded microvessel endothelial cells, such as liposuction fat, with collagenase or combinations of collagenase with other enzymes under conditions which cause the enzymes to disrupt and digest the fatty tissue. Generally, the disruption process does not harm the membranes of the microvessel endothelial cells. By carefully separating the cells from the digested tissue viable microvessel endothelial cells can be isolated.

These viable and intact microvessel endothelial cells have found particular utility as coatings on the interior of synthetic small diameter vascular grafts implanted in humans and animals to replace blood vessels, since they reduce the risk of thrombosis in vascular grafts. Similarly, microvessel endothelial cells are useful as coatings on the surfaces of biomedical implant devices in general, where they provide improved biocompatibility to the implant. Apparently the microvessel endothelial cells contribute to the prevention of undesirable protein deposits and related cellular deposits on the implants, which are known to occur when foreign materials are placed in contact with blood and tissue. In the case of vascular grafts these undesirable deposits can quickly cause the vessel to occlude, resulting in the functional failure of the graft.

Cells which are isolated from animal tissues vary in quality and have varying degrees of viability and efficacy. Even when viable cells are successfully isolated, the yield and degree of viability are frequently unpredictable.

The unpredictable nature of these procedures is attributable to many factors. One factor is the purity of the enzymes or other reagents used in the isolation techniques. Another factor which contributes to the lack of reproducibility in these procedures is the nature of the tissue being digested. While connective tissues are formed largely of collagen, for which collagenase is specific in its hydrolytic activity, significant amounts of other proteins and glycoproteins are additionally found in connective tissue matrices. Mixtures of enzymes are frequently found necessary to effectively dissociate tissues.

Perhaps the key factor in improving the yield of viable microvessel endothelial cells, regardless of which isolation technique is used, however, is the choice of medium in which the cells are placed during isolation and thereafter. For example, cells removed from animal tissue must be stabilized immediately upon their removal if their viability is to be maintained. This is especially important if the cells are intended for use in surgical procedures.

Previously available cell media constituted physiologically compatible liquids of many types. Such physiologically compatible liquids included phosphate buffered saline solutions, such as Dulbecco's phosphate buffered saline and similar electrolyte solutions having osmolarities which are compatible with physiological tissue, such as the commercially available Media 199, which is supplied by Gibco and Sigma. However, these media were not suitable for clinical use. In addition, these media were not completely satisfactory in isolating high yields of viable cells or for stabilizing isolated cells for the periods of time required in using such cells in surgical procedures. Because of the criticality in surgery of maintaining the viability of as many cells as possible for extended periods of time, there is a definite need for providing the optimum environment for cell isolation and stabilization.

Accordingly, it is an object of the present invention to provide a cell medium which maximizes the number of viable cells available for use during surgery.

It is another object of this invention to provide a cell sodding or seeding medium in which the viability of cells is maintained for an extended period of time.

Other objects and advantages of the present invention will become apparent from the following detailed disclosure and description.

Transfusion, vol.31, no.1, 1991, pps 21-25, G Rock *et al*, discloses storage of platelets in "Plasmalyte® electrolyte solution. This solution contains sodium chloride, sodium gluconate, sodium acetate, potassium chloride and magnesium chloride with an osmolarity of 294 mOsmol/l and is commercially available from Baxter Healthcare.

EP-A-446450 discloses the digestion of fat tissue using the enzyme collegenase and isolation of endothelial cells from the digested fat.

The present invention provides a medium for stabilizing viable cells which consists of a mixture of a physiologically acceptable aqueous electrolyte solution of pH 6.5 to pH 8.0 consisting essentially of sodium, potassium, magnesium, chloride, acetate, and gluconate ions, and having an osmolarity of about 270 mOsmol/l to 310 mOsmol/l,and a blood component selected from blood serum and blood plasma, wherein said electrolyte solution and said blood component are present in said mixture in a proportion by weight of from 1:19 to 19:1.

The electrolyte solution may be Plasmalyte®.

The stabilization medium is particularly useful in cell sodding or seeding in vascular grafts where the success of a vascular graft requires coating a layer of viable cells upon the inside surface of the graft. Such medium is also of use in other surgical procedures where prevention of thrombosis is critical, such as in tissue transplants or surgery involving artificial organs.

The absence of typical organic nutrients such as amino acids and vitamins is a decided advantage in sodding or seeding solutions for vascular graft surgery because the blood flowing through the prosthesis inserted during the surgery furnishes its own nutrients, rendering the furnishing of additional nutrients superfluous. Furthermore, the provision of additional organic nutrients may hamper or impede desired enzymatic or other physiological activity. In addition the use of an electrolyte solution which is free of extraneous agents such as nutrients and preservatives ensures that the required cells are present in an optimum environment for viability.

The invention also provides a process for stabilising viable cells isolated from digested animal tissue for a period of up to 4 hours, in which the cells are suspended in the stabilization medium.

The proportion of electrolyte solution to blood component is preferably 3:1 to 7:1 by weight.

Further objects, features, and advantages of the present invention will become apparent to those skilled in the art from a consideration of the following detailed description.

### BRIEF DESCRIPTION OF THE DRAWINGS

FIG. 1 is a plot of the size distribution of cells stabilized in various media.
FIG. 2 is a plot of the size distribution of cells stabilized in various media including the stabilizing medium of the present invention.

### DESCRIPTION OF EXEMPLARY EMBODIMENTS

A preferred process of the present invention utilizes the enzymatic digestion of fatty tissue for dissociation of the fatty tissue and isolation of microvessel endothelial cells embedded therein. Microvessel endothelial cells of high viability are isolated in high yields.

Cell counting techniques provide information relating to cell size and the distribution of cell sizes in a given batch of isolated cells. Cell viability can be demonstrated by the degree of cell proliferation activity which measures the activity of an enzyme present in the cells.

High viability and number of useful cells are particularly important for applications which involve coating the interior wall of synthetic small vessel grafts. This is because the ability of microvessel endothelial cells to prevent protein deposition and subsequent occlusion in the graft is enhanced by the total number and the viability of these cells.

A particularly suitable commercially available electrolyte solution is Plasmalyte® electrolyte solution available from Baxter Healthcare, having a buffered pH of 7.4 and an osmolarity of 294 mOsmol/L obtained with controlled concentrations of sodium, potassium, magnesium, chloride, acetate, and gluconate ions.

For applications directed toward digesting fatty tissue, for example liposuction fat, and isolating cells embedded in the fatty tissue, exemplary compositions which may be used include a solution in the electrolyte solution of an enzyme or mixture of enzymes in an amount sufficient to digest proteinaceous components. For example, collagenase or a mixture of collagenase and chymopapain essentially free of cytotoxins can be used. In a particularly preferred embodiment collagenase in concentrations of about from 10 nKat/ml to 100 nKat/ml and chymopapain in concentrations of about from 0.05 nKat/ml to about 0.5 nKat/ml are used. The nKat/ml unit is defined as nanomoles of substrate hydrolyzed per second by 1 ml of enzyme solution under the assay conditions used. In a most preferred embodiment of the present invention, the enzyme composition is a solution of 50 nKat/ml collagenase and 0.25 nKat/ml chymopapain in Plasmalyte® electrolyte solution.

The enzyme solution is combined with the fatty tissue in about equal parts, for example, at a ratio of 1 ml of enzyme solution to 1 g of fatty tissue, and incubated at about 37° C while shaking until the combination becomes turbid. The desired degree of tissue digestion is thereby achieved.

Further isolation of viable cells from the incubated combination can be accomplished by centrifuging the incubated combination until adipocytes and a supernatant separate from the combination and microvessel endothelial cells form as a pellet. The cell pellet can be removed from the separated combination by pipetting off the adipocytes and supernatant. Isolated cells are thus provided in the form of a pellet. Preferred exemplary processes further include rinsing the cell pellet with a physiologically compatible liquid and then centrifuging the rinsed cell pellet prior to the evaluation and use of the cells.

Fatty tissues derived from liposuction which are subject to digestion should preferably be substantially homogeneous and without apparent large pieces of fat. Accordingly, nonhomogeneous appearing liposuction fatty mixtures can be minced in a tissue mincer prior to further treatment. Additionally, the liposuction fatty mixtures are preferably rinsed with a suitable physiologically compatible rinsing solution in order to remove visual blood contaminants including clotted blood. A preferred method for rinsing the mixture involves transferring the liposuction fatty mixture into a plastic inner basket or sieve-tissue grinder cup and adding phosphate buffered saline solution to the mixture while stirring. Excess liquids and blood contaminants are removed by the rinsing and sieving process. The homogenized and rinsed liposuction fatty mixture is then prepared for the above-described digestion and microvessel cell isolation procedures.

As generally mentioned above, microvessel endothelial cells isolated from liposuction fatty tissues and other cell types isolated from various animal tissues are isolated in higher yields and have greater viability than cells isolated by prior art processes.

In accordance with the present invention, the viability of the cells is considerably enhanced by suspending the isolated cells in a mixture of the electrolyte solution with a blood component, which is blood serum or blood plasma. For use in surgery involving human beings, human blood components are preferred. As a matter of convenience the blood serum or plasma of the patient undergoing the vascular graft typically is used. Cell viabilities of up to about 4 hours can be realized.

The superior physical and functional characteristics of the cells isolated are demonstrated by the higher number of cells having preferred sizes as determined by known cell counting methods. Other indicators of superior results include the improved adhesion capabilities and cell proliferation activities of the isolated cells. The improved ability of cells to adhere to surfaces and to freely proliferate demonstrates their improved viability. Similarly, since intact and viable microvessel cells are larger than 7.78µ, a higher number of these larger cells is an indicator of a highly efficacious process.

The large particle diameter material is known to be highly viable metabolically active cells. In contrast small particle size materials include cellular material and tissue debris having little therapeutic usefulness.

The stabilization process of the present invention, which consists of suspending isolated cells in the stabilizing medium of the present invention, that is, a mixture of the electrolyte solution with serum or plasma, results in enhancing and prolonging the viability of the isolated cells. The stabilizing effect of the process of the present invention is demonstrated by counting techniques, such as the use of a Coulter Multisizer to show the higher number of viable cells observed using the stabilizing medium of the present invention.

In addition, cell adhesion studies performed by incubating a fixed number of cells on cell culture plates having a coating of cell adhesion molecules, washing the plates to remove unattached cells and then visualizing the density of attached cells further demonstrates the improved stability and integrity of cells stabilized according to the teachings of the present invention, which produce densely covered areas of attached cells on the cell culture plates. As known in the art, this is indicative of highly viable cells with active surface receptors for adhesion materials.

Finally, cell proliferation studies of cells stabilized according to the present invention further demonstrate their enhanced integrity. Cells which have been stabilized according to the present invention and then allowed to proliferate have a higher enzyme activity, an indication of the presence of more metabolically active cells, than those isolated according to prior art procedures. These results clearly demonstrate that cells treated in accordance with the teachings of the present invention have greater functional integrity than comparably treated cells using prior art compositions and methodologies.

The resultant superior physical and functional characteristics of cells isolated make them particularly useful in surgery. Microvessel endothelial cells, for example, are useful as coatings for the interior walls of small diameter artificial blood vessel grafts. These grafts are typically fabricated of expanded polytetrafluoroethylene, dacron, polyurethane, polyurethane urea, or combinations thereof and implanted in humans and other mammals as artificial blood vessels. When coated on the interior surface of these artificial grafts according to methods known in the art, the increased population and better viability of the microvessel endothelial cells provided by the present invention make it easier for the cells to effectively adhere to the interior walls of the graft with thorough coverage and to maintain their functional integrity. The high viability and functional ability of these cells provide a graft surface that is less prone to protein and related cellular deposition. Accordingly, grafts treated according to the teachings of the present invention are less susceptible to functional failure such as occlusion caused by undesirable protein and cellular deposits. In addition, their use prevents thrombosis during surgery.

The following nonlimiting examples illustrate exemplary compositions of the present invention. The examples also demonstrate the improved stability of cells when maintained in the stabilization medium of the present invention.

### EXAMPLE 1

Rabbit fat was digested in collagenase solutions using either 4 mg/ml collagenase in Dulbecco's phosphate buffered saline or 4 mg/ml collagenase in a mixture of Plasmalyte® electrolyte solution and 4 mg/ml human serum albumin. For the latter solution 0.4 g collagenase and 1.6 ml human serum albumin solution were mixed in 100 ml Plasmalyte® electrolyte solution of pH 7.4 and 294 mOsmol/L osmolarity. Plasmalyte® electrolyte solution has the composition shown in Table 1.

**TABLE 1**

| | |
|---|---|
| Sodium Chloride | 5.26 g/L |
| Sodium Gluconate | 5.02 g/L |
| Sodium Acetate, Trihydrate | 3.68 g/L |
| Potassium Chloride | 0.37 g/L |
| Magnesium Chloride | 0.3 g/L |

Digestion was accomplished in a 37°C water bath (Precision Scientific Dubnoff Metabolic Shaker), incubated for 20 minutes at 100 cycles/min. A pellet was produced by centrifuging at 1800 rpm for 7 minutes. The pellet was then resuspended in 0.1% bovine albumin in Dulbecco's phosphate buffered saline, centrifuged again at 1800 rpm for 4 min. and the cells were counted using a Coulter Multisizer II. The cell yield isolated from Dulbecco's phosphate buffered saline was 5.98 x 10⁵ cells per gram of fat. The cell yield isolated from Plasmalyte® electrolyte solution was 7.38 x 10⁵.

The results of this experiment clearly show that Plasmalyte® electrolyte solution was a more effective isolation medium than Dulbecco's phosphate buffered saline.

The following example illustrates the stability of cells isolated from human liposuction fat in Plasmalyte® electrolyte solution compared with the stability of cells in a typical cell sodding medium.

### EXAMPLE 2

Human liposuction fat was obtained 45 to 90 minutes previous to the experiment. The fat was rinsed with Dulbecco's phosphate buffered saline and drained. Ten (10) grams of fat and 10 g of collagenase solution were added to flasks and placed in a shaker incubator at 37 C for 20 min at 100 cycles/min.

The resulting slurry was pooled and poured into 15 ml conical centrifuge tubes and spun at 1800 rpm for seven minutes.

The endothelial cells and red blood cells precipitated at the bottom. Dark collagenase solution formed a middle layer and fat formed a plug on top of the centrifuge tube. Both the dark collagenase solution and the fat were discarded. The endothelial cell pellets were resuspended at 0.1% bovine albumin in Dulbecco's phosphate buffered saline, pooled in a new sterile conical centrifuge tube and spun at 1800 rpm for 4 minutes. The supernatant was discarded and the endothelial cells pellet was resuspended with 14% human serum in Media 199 supplied by Gibco. The cell solution was equally divided into two centrifuge tubes and spun at 1800 rpm for an additional 4 minutes. The supernatant was discarded. One endothelial cell pellet was resuspended with Plasmalyte® electrolyte solution and the other endothelial cell pellet was resuspended with Medium 199/serum. The volume of each was 5 ml. The 0.5 ml endothelial cell suspension was diluted to 20 ml with Isoton solution and the cell yield and size determined using a Coulter Multisizer II.

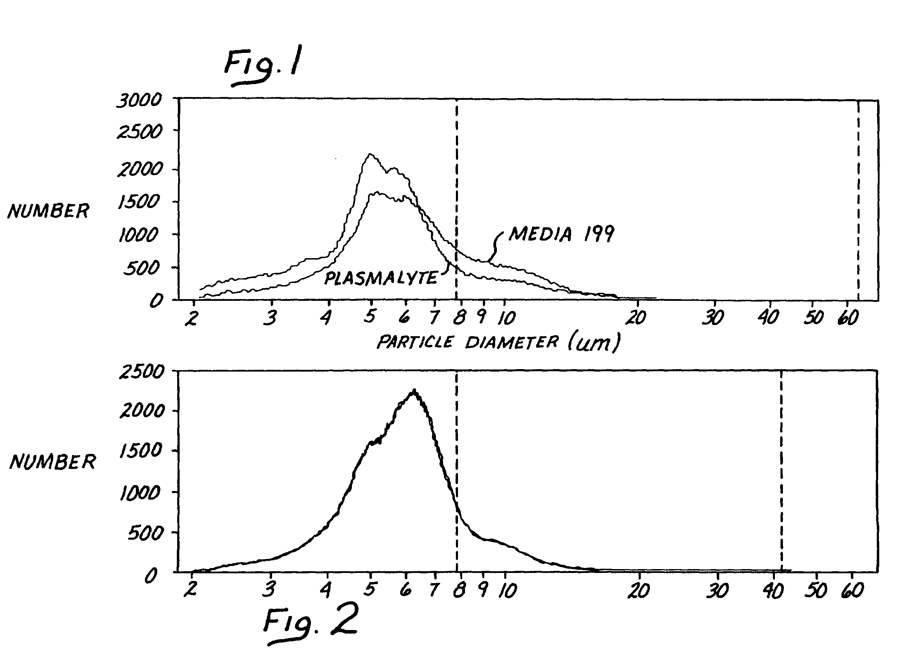

The results are shown in FIG. 1.

It can be seen from the plots shown in FIG. 1 that Media 199/serum is a more effective stabilizing medium than Plasmalyte® electrolyte solution.

The following example illustrates the stability of cells isolated from human liposuction fat in Plasmalyte® electrolyte solution/serum compared with the stability of cells in Media 199/serum.

### EXAMPLE 3

The procedure of Example 2 was followed except that Plasmalyte® electrolyte solution/serum (14% human serum) was substituted for Plasmalyte® electrolyte solution. The results are shown in FIG. 2.

It can be seen from the plots shown in FIG. 2 (the two plots are essentially superimposed) that Plasmalyte® electrolyte solution/serum, the stabilizing medium of the present invention, is at least as effective a stabilizing medium as Media 199/serum, a typical prior art medium.

The following example illustrates the long term stability of cells stabilized in Plasmalyte® electrolyte solution/serum compared with the stability of cells stabilized in Media 199/serum.

### EXAMPLE 4

The procedure of Example 3 was followed. The number of isolated cells of diameter > 7.78 microns per 500 microliters of solution was determined after 2 hours of storage at room temperature utilizing either Plasmalyte® electrolyte solution/serum or Media 199/serum. The results are shown in Table 2.

**TABLE 2**

| Plasmalyte® electrolyte solution/serum | Media 199/serum |
|---|---|
| 13969 ± 151 | 12954 ± 200 |

These results show that Plasmalyte® electrolyte solution/serum is more effective than Media 199/serum in stabilizing cells after 2 hours storage.

## Claims

1. A medium for stabilizing viable cells which consists of a mixture of:
a physiologically acceptable aqueous electrolyte solution pH 6.5 to pH 8.0 consisting essentially of sodium, potassium, magnesium, chloride, acetate, and gluconate ions, and having an osmolarity of about 270 mOsmol/l to 310 mOsmol/l; and
a blood component selected from blood serum and blood plasma,
wherein said electrolyte solution and said blood component are present in said mixture in a proportion by weight of from 1:19 to 19:1.

2. A medium according to Claim 1, wherein said electrolyte solution and said blood component are present in said mixture in a proportion by weight of about from 3:1 to 7:1.

3. A medium according to Claim 3 wherein said blood component is human blood serum.

4. A process for stabilizing viable cells isolated from digested animal tissue for a period of up to 4 hours which comprises suspending said cells in the medium of any one of Claims 1 to 3.

5. A process according to Claim 4, wherein said animal tissue is human tissue.

6. A process according to Claim 4 or 5, including isolating viable endothelial cells from tissue containing same prior to stabilizing the cells, by providing an electrolyte solution of pH 6.5 to pH 8.0 consisting essentially of sodium, potassium, magnesium, chloride, acetate, and gluconate ions, and having an osmolarity of about 270 mOsmol/l to 310 mOsmol/l, and containing an enzyme composition in an amount sufficient to digest said tissue;
contacting said tissue with said solution for a length of time and at a temperature sufficient to substantially digest said tissue while leaving intact the viable cells contained within said tissue; and
separating viable cells from the digested tissue.

7. A process according to Claim 6, wherein said cells are microvessel endothelial cells.

8. A process according to Claim 6, wherein said tissue is fatty tissue.

9. A process according to any one of Claims 6 to 8, wherein said enzyme composition comprises a member selected from collagenase, dispase, trypsin, elastase, papain, chymopapain, pronase, hyaluronidase and mixtures thereof.

10. A process according to any one of Claims 6 to 9, followed by coating the inner wall surface of an elongated generally tubular physiologically compatible membrane with the separated viable cells to produce a vascular graft having improved resistance to occlusion.

11. A process according to Claim 10, wherein said membrane is fabricated from polytetrafluoroethylene, dacron, polyurethane, polyurethane urea, or combinations thereof.

## Patentansprüche

1. Medium zum Stabilisieren von lebensfähigen Zellen, bestehend aus einer Mischung aus:
- einer physiologisch verträglichen wässerigen Elektrolytlösung mit einem pH-Wert von 6,5 bis 8,0, die im wesentlichen aus Natrium-, Kalium-, Magnesium-, Chlorid-, Acetat- und Gluconationen besteht und die eine Osmolarität von ca. 270 mOsmol/l bis 310 mOsmol/l aufweist; und
- einer Blutkomponente, ausgewählt aus Blutserum und Blutplasma,
- wobei die Elektrolytlösung und die Blutkomponente in der Mischung in einem Gewichtsanteil von 1:19 bis 19:1 vorhanden sind.

2. Medium nach Anspruch 1,
wobei die Elektrolytlösung und die Blutkomponente in der Mischung in einem Gewichtsanteil von ca. 3:1 bis 7:1 vorhanden sind.

3. Medium nach Anspruch 3,
wobei die Blutkomponente menschliches Blutserum ist.

4. Verfahren zum Stabilisieren lebensfähiger Zellen, die aus aufgeschlossenem tierischem Gewebe während eines Zeitraums von bis zu 4 Stunden isoliert wurden, welches das Suspendieren der Zellen in dem Medium nach einem der Ansprüche 1 bis 3 umfaßt.

5. Verfahren nach Anspruch 4,
wobei das Tiergewebe ein menschliches Gewebe ist.

6. Verfahren nach Anspruch 4 oder 5,
umfassend das Isolieren lebensfähiger Endothelialzellen aus Gewebe, die dieses vor der Stabilisiertang der Zellen enthält, das folgende Schritte aufweist:
- Bereitstellen einer Elektrolytlösung mit einem pH-Wert von 6,5 bis 8,0, die im wesentlichen aus Natrium-, Kalium-, Magnesium-, Chlorid-, Acetat- und Gluconationen besteht und die eine Osmolarität von ca. 270 mOsmol/l bis 310 mOsmol/l besitzt und die eine Enzymzusammensetzung in einer Menge enthält, um das Gewebe aufzuschließen;
- in-Kontakt-bringen des Gewebes mit der Lösung während einer Zeitdauer und einer Temperatur, die ausreicht, um das Gewebe im wesentlichen aufzuschließen, während die im Gewebe enthaltenen lebensfähigen Zellen intakt bleiben; und
- Abtrennen der lebensfähigen Zellen vom aufgeschlossenen Gewebe.

7. Verfahren nach Anspruch 6,
wobei die Zellen Mikroblutgefäß-Endothelialzellen sind.

8. Verfahren nach Anspruch 6,
wobei das Gewebe Fettgewebe ist.

9. Verfahren nach einem der Ansprüche 6 bis 8,
wobei die Enzymzusammensetzung eine Substanz enthält, die aus Kollagenase, Dispase, Trypsin, Elastase, Papain, Chymopapain, Pronase, Hyaluronidase und Mischungen davon ausgewählt ist.

10. Verfahren nach einem der Ansprüche 6 bis 9,
bei dem man anschließend die innere Wandoberfläche einer länglichen im wesentlichen rohrförmigen physiologisch kompatiblen Membran mit den abgetrennten Lebensfähigen Zellen beschichtet, um ein Gefäßtransplantat mit einer verbesserten Widerstandsfähigkeit gegenüber Okklusion herzustellen.

11. Verfahren nach Anspruch 10,
wobei die Membran aus Polytetrafluorothylen, Dacron, Polyurethan, Polyurethanharnstoff oder Kombinationen davon hergestellt wird.

## Revendications

1. Milieu pour stabiliser des cellules vivantes, qui est constitué d'un mélange :
d'une solution électrolytique aqueuse acceptable d'un point de vue physiologique, ayant un pH de 6,5 à 8,0, constituée essentiellement d'ions sodium, potassium, magnésium, chlorure, acétate et gluconate, et ayant une osmolarité d'environ 270 mOsmol/l à 310 mOsmol/l ; et
d'un composant du sang choisi parmi le sérum sanguin et le plasma sanguin,
où ladite solution électrolytique et ledit composant sanguin sont présents dans ledit mélange selon une proportion on poids de 1:19 à 19:1.

2. Milieu selon la revendication 1, dans lequel ladite solution électrolytique et ledit composant sanguin sont présents dans ledit mélange selon une proportion en poids d'environ 3:1 à 7:1.

3. Milieu selon la revendication 3, dans lequel ledit composant sanguin est le sérum sanguin humain.

4. Procédé de stabilisation do cellules viables isolées d'un tissu animal digéré, pendant une période allant jusqu'à 4 heures, qui consiste à mettre en suspension lesdites cellules dans le milieu selon l'une quelconque des revendications 1 à 3.

5. Procédé selon la revendication 4, dans lequel ledit tissu animal est un tissu humain.

6. Procédé selon la revendication 4 ou 5, qui consiste à isoler des cellules endothéliales viables d'un tissu contenant ces dernières, avant stabilisation des cellules, en mettant à disposition une solution électrolytique ayant un pH de 6,5 à 8,0, constituée essentiellement d'ions sodium, potassium, magnésium, chlorure, acétate et gluconate et ayant une osmolarité d'environ 270 mOsmol/l à 310 mOsmol/l, et contenant une composition enzymatique en une quantité suffisante pour digérer ledit tissu ;
à mettre on contact ledit tissu avec ladite solution pendant un laps de temps et à une température suffisants pour provoquer la digestion presque complète dudit tissu, tout on laissant intactes les cellules viables contenues dans ledit tissu ; et
à séparer les cellules viables du tissu digéré.

7. Procédé selon la revendication 6, dans lequel lesdites cellules sont des cellules endothéliales de microvaisseaux.

8. Procédé selon la revendication 6, dans lequel ledit tissu est un tissu gras.

9. Procédé selon l'une quelconque des revendications 6 à 8, dans lequel la composition enzymatique comprend un membre de l'ensemble comprenant la collagénase, la dispase, la trypsine, l'élastase, la papaïne, la chymopapaïne, la pronase, l'hyaluronidase et leurs mélanges.

10. Procédé selon l'une quelconque des revendications 6 à 9, qui se poursuit par l'application, sur la surface de paroi intérieure d'une membrane allongée et généralement tubulaire, compatible d'un point de vue physiologique, des cellules viables séparées, dans le but de produire une greffe vasculaire ayant ne résistance accrue à l'occlusion.

11. Procédé selon la revendication 10, dans lequel ladite membrane est fabriquée à partir de polytétrafluoréthylène, de dacron, de polyuréthanne, de polyuréthanne-urée ou de leurs combinaisons.
